# EUROPEAN PATENT SPECIFICATION

(11) **EP 3 533 451 B1**
(45) Date of publication and mention of the grant of the patent: **27.07.2022**
(21) Application number: 17892917.0
(22) Date of filing: 14.02.2017
(51) Int. Cl.: A61K 31/7048, A61P 27/02, A61P 37/00, A61K 45/06

(54) **APPLICATION OF PAEONIFLORIN-6'-O-BENZENE SULFONATE IN MEDICINE FOR TREATING SJÖGREN'S SYNDROME**
VERWENDUNG VON PAEONIFLORIN-6'-O-BENZOLSULFONAT IN DER MEDIZIN ZUR BEHANDLUNG VON SJÖGREN-SYNDROM
APPLICATION DE PAEÉNIFLORIN-6'-O-BENZÈNE SULFONATE EN MÉDECINE POUR LE TRAITEMENT DU SYNDROME DE SJÖGREN

(30) Priority: 21.01.2017 WO PCT/CN2017/071998
(43) Date of publication of application: 04.09.2019
(73) Proprietor: Guangzhou Hanfang Pharmaceuticals Co., Ltd., Guangzhou Guangdong 510970 (CN)
(72) Inventor: WEI, Wei, Ningbo Zhejiang 315194 (CN); WU, Huaxun, Ningbo Zhejiang 315194 (CN); GU, Fang, Ningbo Zhejiang 315194 (CN); XU, Jun, Ningbo Zhejiang 315194 (CN); XIE, Hongwei, Ningbo Zhejiang 315194 (CN); XU, Guang, Ningbo Zhejiang 315194 (CN); FU, Jie, Ningbo Zhejiang 315194 (CN); WANG, Xuezheng, Ningbo Zhejiang 315194 (CN)
(74) Representative: Gulde & Partner
(86) International application number: PCT/CN2017/073540
(87) International publication number: WO 2018/133139

(56) References cited:
- WO-A1-2004/108216
- CN-A- 102 603 827
- LI CHUN LEI ET AL: "Effects of total glucosides of paeony for delaying onset of Sjogren's syndrome: an animal study.", JOURNAL OF CRANIO-MAXILLO-FACIAL SURGERY : OFFICIAL PUBLICATION OF THE EUROPEAN ASSOCIATION FOR CRANIO-MAXILLO-FACIAL SURGERY OCT 2013, vol. 41, no. 7, October 2013 (2013-10), pages 610-615, XP009517615, ISSN: 1878-4119
- YU JINGYA ET AL: "Paeoniflorin down-regulates ATP-induced inflammatory cytokine production and P2X7R expression on peripheral blood mononuclear cells from patients with primary Sjogren's syndrome", INTERNATIONAL IMMUNOPHARMACOLOGY, vol. 28, no. 1, September 2015 (2015-09), pages 115-120, XP009517617, ISSN: 1567-5769
- JIA XIAOYI ET AL: "CP-25 attenuates the inflammatory response of fibroblast-like synoviocytes co-cultured with BAFF-activated CD4+T cells", JOURNAL OF ETHNOPHARMACOLOGY, ELSEVIER IRELAND LTD, IE, vol. 189, 16 May 2016 (2016-05-16), pages 194-201, XP029683670, ISSN: 0378-8741, DOI: 10.1016/J.JEP.2016.05.034

## Description

### Technical Field

The present invention relates to the field of pharmaceutical, in particular to the use of Paeoniflorin-6'-O-benzenesulfonate in the treatment of Sjögren's syndrome.

### Background Art

Sjögren's syndrome (SS) is an autoimmune disorder characterized mainly in that the immune cells attack and destroy the glands that produce tears and saliva. The main pathological features of SS are B lymphocyte hyperfunction and suppressor T lymphocytes dysfunction. SS is a multifactorial disease, which may be related to genetic environment, viral infection and immunological factors, etc. Primary Sjögren's syndrome (pSS) is a global disease, and affects 0.33%∼0.77% of individuals in China, which mainly occurs in women and the elderly and has the ratio of about 1: 9∼1: 20 of male to female. The hallmark symptoms of the disorder are dry mouth and dry eyes, and some patients experience intermittent mumps and following rampant caries. In addition, pSS may also cause other atypical general manifestation and systemic impairment, for example, relatively common joint pain, kidney injury and lung disease, etc.

There are two main types of drug for clinical treatment of SS, i.e., disease relief drug and systemic therapeutic drug. The replacement therapies of artificial saliva or artificial tears could relieve the symptoms of local dryness; M3 receptor agonists, for example, Pilocarpine or Cevimeline could promote secretion of saliva and tears and in turn fairly significantly ameliorate patient's symptoms of mouth and eye dryness by stimulating the receptors in salivary and lacrimal glands; appropriate dose of glucocorticosteroid could be used to protect damaged tissues and organs, and reduce systemic inflammatory response; immunosuppressive agents, for example, hydroxychloroquine (HCQ) could inhibit inflammatory immune response, relieve the infiltration of exocrine gland, and protect the injured exocrine gland and organ function, etc.

Artificial replacement such as artificial saliva could hardly maintain the dry and clean environment of mouth for a long time, while the therapy of M3 receptor agonists just partly relieve the symptoms of exocrine gland, and leave alone that the immunosuppressive agents cause various side effects when suppressing the immune response. Therefore, aforementioned drugs including promoting treatment of Sjögren's syndrome and alternative therapeutic drugs could not fundamentally cure the organ damage caused by the disease and various side effects induced by immunosuppressive agents. In brief, there exists urgent demand for the clinical drug with satisfactory effects and less adverse reaction.

In WO 2004/108216 A1, Li Chun Lei et al.: "Effects of total glucosides of paeony for delaying onset of Sjörgen's syndrome: an animal study"; Journal of Cranio-Maxillo-Facial Surgery, 2013, vol. 41, pages 610-615, and Yu Jingya et al.: "Paeoniflorin down-regulates ATP-induced inflammatory cytokine production and P2X7R expression on peripheral blood mononuclear cells from patients with primary Sjörgen's syndrome"; International Immunopharmacology, 2015, vol. 28, pages 115-120, it is disclosed that total glucosides of paeony (TGP) and in particular its constituent paeoniflorin can be used in treatment of Sjörgen's syndrome.

In Jia Xiaoyi et al.: "CP-25 attenuates the inflammatory response of fibroblast-like synoviocytes co-cultured with BAFF-activated CD4+T cells"; Journal of Ethnopharmacology, 2016, vol. 189, pages 194-201, it is shown that a derivative of paeoniflorin, namely paeoniflorin-6'-O-benzene sulfonate (CP-25), has improved intestinal absorption parameters as compared to paeoniflorin.

### Summary of the invention

The present invention is directed to subject matter as defined in the claims. In particular, the present invention is directed to Paeoniflorin-6'-O-benzene sulfonate (code-named CP-25) for use in increasing saliva flow in mammalian Sjörgen's syndrome.

The present invention aims at provision of a substance which could be used for treatment of Sjögren's syndrome. The substance, Paeoniflorin-6'-O-benzene sulfonate (code-named CP-25), which is obtained from structural modification of Paeoniflorin (Pae) extracted from *Paeonia lactiflora Pall.,* is superior to Total Glucosides of Paeony or Pae, on onset time and action intensity of anti-inflammatory effects.

The molecular weight and molecular formula of paeoniflorin-6'-O-benzenesulfonate are 620.62 and C₂₉H₃₂O₁₃S respectively, and the chemical structure is shown in Formula I.

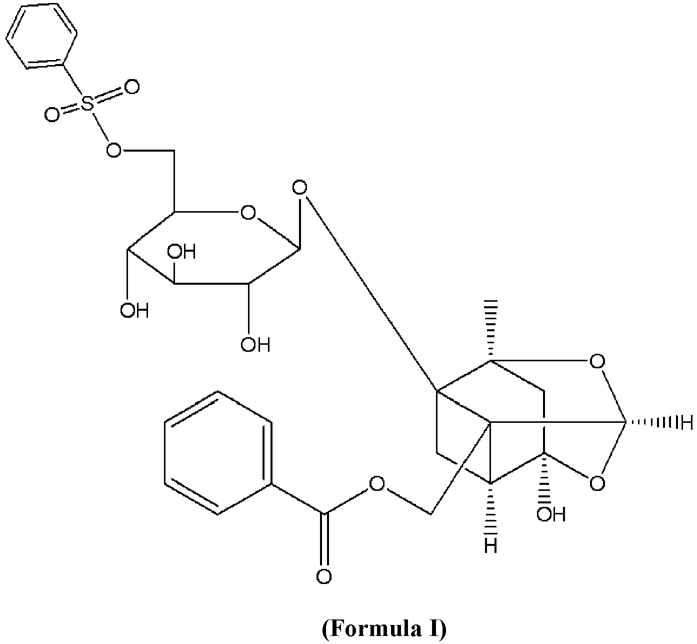

In first aspect, the present invention relates to use of paeoniflorin-6'-O-benzenesulfonate in preparation of a pharmaceutical agent used for the treatment of mammalian Sjögren's syndrome.

In second aspect, the present invention relates to use in the first aspect thereof characterized in that the effective amount of paeoniflorin-6'-O-benzenesulfonate ranges from 17.5mg/kg to 70mg/kg.

In third aspect, the present invention relates to use in the first aspect thereof characterized in that the effective amount of paeoniflorin-6'-O-benzenesulfonate ranges from 17.5mg/kg to 35mg/kg.

In fourth aspect, the present invention relates to use in the first aspect thereof characterized in that the effective amount of paeoniflorin-6'-O-benzenesulfonate is 17.5mg/kg.

In fifth aspect, the present invention relates to use in any one of aspects 1 to 4 thereof, wherein said pharmaceutical agent is a drug combination comprising paeoniflorin-6'-O-benzenesulfonate and a further therapeutic agent.

In sixth aspect, the present invention relates to use in the fifth aspect thereof, wherein the further therapeutic agent is one or more members selected from the group consisting of: a drug for autoimmune diseases, a further drug for the treatment of Sjögren's syndrome, and a drug for promoting treatment of Sjögren's syndrome.

In seventh aspect, the present invention relates to use in the sixth aspect thereof, wherein said drug for autoimmune diseases is one or more members selected from the group consisting of: biological agents acting on TNF-α target, for example, Humira, Enbrel, Remicade, Simponi (Golimumab), Cimzia (Certolizumab pegol), Etanercept (Recombinant human tumor necrosis factor-α receptor II: IgG Fc fusion protein for injection); other biological agents for treatment of autoimmune diseases, for example, Actemra (Ocilizumab), Orencia (Abatacept), Stelara (Ustekinumab); JAK inhibitors acting on JAK1, JAK2, JAK3 or TYK2 target, etc., for example, Xeljanz (Tofacitinib), Baricitinib; selective phosphodiesterase 4 (PDE4) inhibitors, for example, Otezla (Apremilast).

In eighth aspect, the present invention relates to use in the sixth aspect thereof, wherein the further drug for the treatment of Sjögren's syndrome is one or more members selected from the group consisting of: drugs for relieving local dryness symptom, for example, Pilocarpine, Ciclosporin, Anethol Trithione, Cevimeline, artificial tears; immunosuppressive agents for systemic therapy, for example, Methotrexate, Leflunomide, Cyclophosphamide, Azathioprine, Tripterygium wilfordii, Tacrolimus, Mycophenolate mofetil; glucocorticoids, for example, Prednisone, Methylprednisolone; and other drugs for treatment of autoimmune diseases, for example, Hydroxychloroquine, Sulfasalazine, anti CD20 antibody.

In ninth aspect, the present invention relates to use in the sixth aspect thereof, wherein the drug for promoting treatment of Sjögren's syndrome is one or more members selected from the group consisting of the substances described in the following patent/application:
WO2016204429 (A1), CA2950893 (A1), KR20160126734 (A), HK1216994 (A1), US2016362462 (A1), US2016361407 (A1), US2016361360 (A1), US2016361428 (A1), US2016361300 (A1), WO2016200447 (A1), CA2950423 (A1), US2016348072 (A1), US2015065352 (A1), US9512487 (B2), US2016356788 (A1), US2016355591 (A1), US2016355464 (A1), US2016354437 (A1), TW201632559 (A), TW201630938 (A), TW201630880 (A), WO2016196429 (A1), WO2016196393 (A2), MD20160069 (A2), CA2949966 (A1), US2016346328 (A1), US2016346355 (A1), US2016345547 (A1), NZ702458 (A), WO2016191545 (A1), WO2016190630 (A1), WO2016191634 (A1), AU2015253915 (A1), AU2015244025 (A1), AU2015254818 (A1), AU2015254037 (A1), US2016339053 (A1), US2016339049 (A1), US2016340337 (A1), US2016338984 (A1), US2016338953 (A1), WO2016185476 (A1), HRP20161191 (T1), RU2601410 (C1), US2016333089 (A1), US2016333103 (A1), US2016333409 (A1), US2016331834 (A1), US2016331816 (A1), AU2016250372 (A1), AU2016247182 (A1).

In tenth aspect, the present invention relates to use in the fifth aspect thereof, wherein said drug combination could be administrated with a composition comprising paeoniflorin-6'-O-benzenesulfonate and the further therapeutic agent, or by respectively administering paeoniflorin-6'-O-benzenesulfonate and the further therapeutic agent, simultaneously or sequentially.

In this invention, low dose (17.5mg/kg), medium dose (35mg/kg) and high dose (70mg/kg) of CP-25 were respectively administrated by gavage to each of randomly assigned group of Sjögren's syndrome model mice for two weeks. Water intake, saliva amount and submaxillary gland pathological analysis and score were determined respectively, on the day before administration, the 7^{th} day and 14^{th} day after administration. The therapeutic effects of CP-25 on Sjögren's syndrome were evaluated by comparison of changes of each index before and after treatment.

### Animal experiments:

Sjögren's syndrome model mice: C57BL/6 female mice housed in a SPF environment with successful modeling (The animal model was made via repeated multi-point intracutaneous injection of submaxillary gland antigen of homologous mice to tail and back of mice).

### Construction of model and grouping:

The animal model was made via repeated multi-point intracutaneous injection of submaxillary gland homogenate of homologous mice to tail and back of mice, and the mice with successful modeling were randomly assigned to each group according to comprehensive conditions of mice. At the same time, the average weight and status of each group of mice should be as similar as possible.

### Preparation and administration of paeoniflorin-6'-O-benzenesulfonate:

Paeonifloiin-6'-O-benzenesulfonate, white crystalline powder, purity 98.5%, was dissolved in trace ethanol (75 mg of CP-25 was dissolved in 40 µl of ethanol), and then sodium carboxymethyl cellulose was added to formulate a suspension of CP-25. Corresponding dose gradient, low dose (17.5 mg/kg), medium dose (35 mg/kg) and high dose (70 mg/kg) of CP-25 should be prepared before medication, stored in 4°C refrigerator for use. High dose of CP-25 should be incubated for half an hour before use.

### Measurement of water intake:

Water intake in therapeutic mice tends to be increased before medication, but begins to drop off after a week of administration with CP-25. However, the water intake in model control group of mice always tends to rise.

### Measurement of saliva amount:

Before measurement, mice were anesthetized via intraperitoneal injection of 0.05 ml of 2.4% Pentobarbital sodium, and the satisfactory anesthesia was smooth breathing, corneal reflex disappearance and limb muscle relaxation. After complete anesthesia, each mouse was placed in slightly tilted trendelenburg position, and its body was kept warm using the heater. The mouse was injected intraperitoneally with 0.1 ml of 0.025 mg/mL Pilocarpine, and 5 min later, a weighed cotton ball was placed in the mouth of the mouse for 10 min. After taking the ball out, the saliva weight of mice was measured by the wet weight method. Saliva amount in model group of mice was apparently decreased, however, that in therapeutic group of mice was obviously recovered after one and two weeks of administration.

### Histopathological examination of submaxillary gland:

Mice were sacrificed after two weeks of administration, and then submaxillary glands of four mice of each group were removed and pathologically observed. At first, the submaxillary gland should be fixed in 10% buffered formalin for 24 h, followed by washing, dehydration and clearing, wax-filling, embedding, sectioning and finally H&E staining. There were varying degrees and ingravescence of lymphocyte infiltration in submaxillary glands in model mice; on the contrary, described infiltration could be significantly improved in therapeutic group mice. Results were graded pathologically.

### Advantages of this invention are:

The present inventors have unexpectedly found that, paeoniflorin-6'-O-benzenesulfonate of the invention has the same or even better curative effects on Sjögren's syndrome when administrated with dose much lower than conventional dose, so that it could greatly decrease adverse reaction, significantly improve patient compliance, and then get superior therapeutic effects.

### Description of drawings

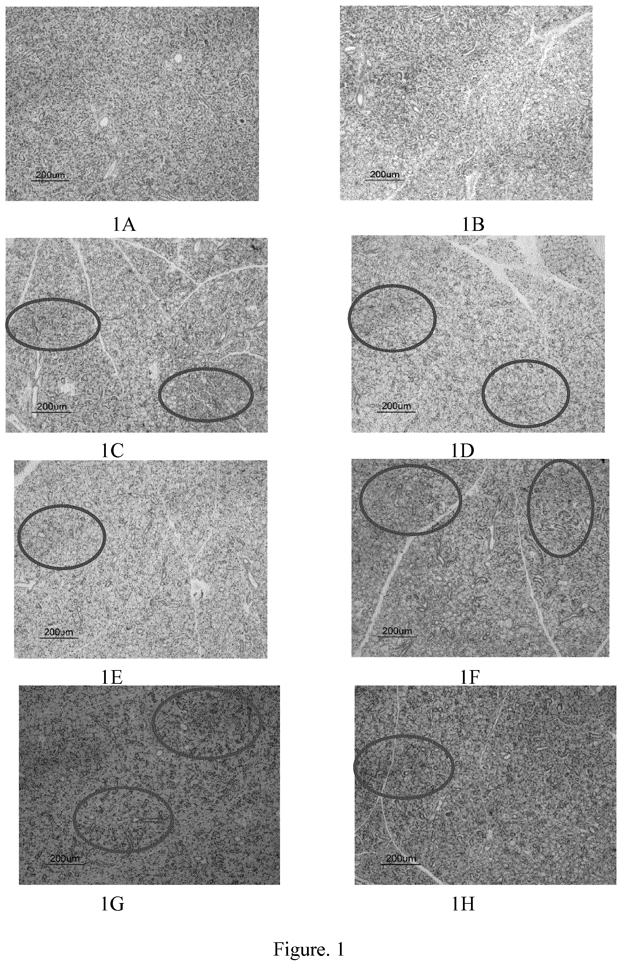

Fig.1 Effects of CP-25 on pathology of submaxillary gland in SS model mice (HE× 100). A: Control; B: Model; C: Low dose of CP-25; D: Medium dose of CP-25; E: High dose of CP-25; F: TGP; G: Pae; H: HCQ.

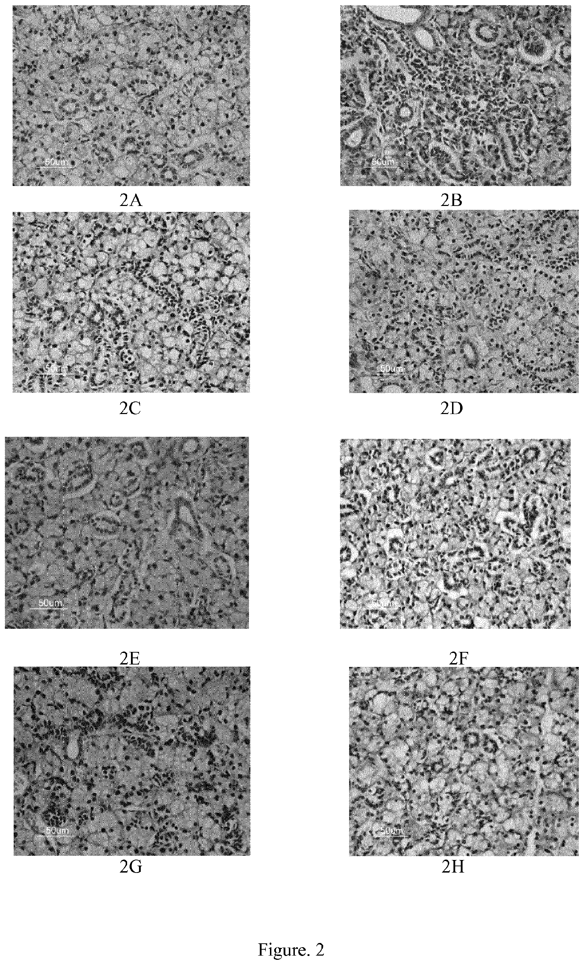

Fig.2 Effects of CP-25 on pathology of submaxillary gland in SS model mice (HE×400). A: Control; B: Model; C: Low dose of CP-25; D: Medium dose of CP-25; E: High dose of CP-25; F: TGP; G: Pae; H: HCQ.

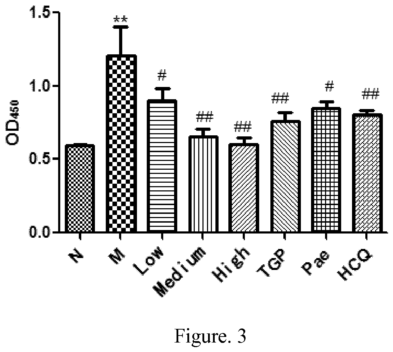

Fig.3 Effects of CP-25 on spleen B lymphocyte proliferation in SS model mice. ^{∗∗}*P*<0.01 vs. Normal; # *P*<0.05, # # *P*<0.01 vs. Model
   The results of Fig.3 showed that CP-25 could significantly inhibit B lymphocyte proliferation in SS model mice.

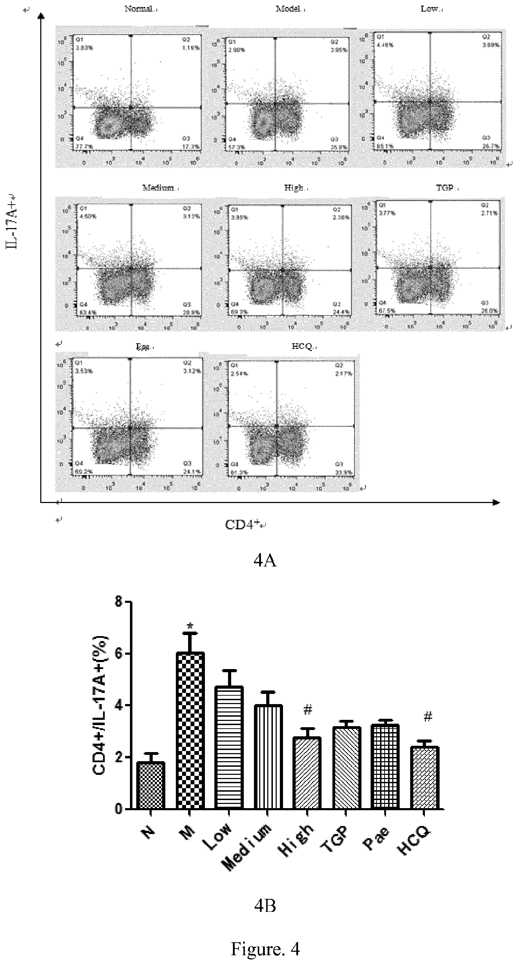

Fig.4 Effects of CP-25 on spleen CD4⁺ IL-17A⁺ T lymphocytes in SS model mice. ^{∗}*P*<0.05 vs. Normal; # *P*<0.05 vs. Model
   The results of Fig.4 showed that CP-25 could significantly inhibit the ratio of CD4⁺ IL-17A⁺ Th17 lymphocytes in SS model mice.

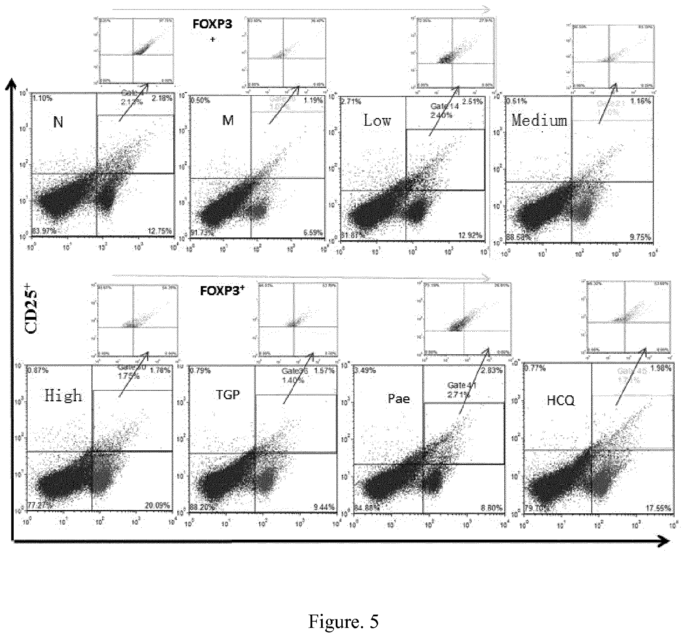

Fig.5 Effects of CP-25 on spleen CD4⁺ CD25⁺FOXP3⁺ T lymphocytes in SS model mice.

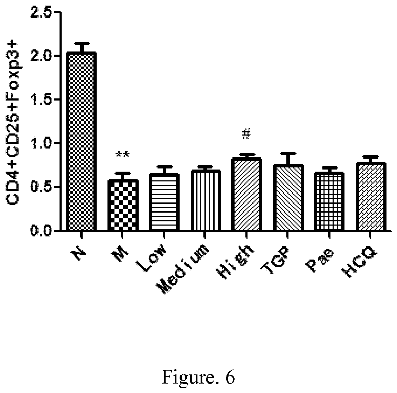

Fig.6 Effects of CP-25 on spleen CD4⁺ CD25⁺FOXP3⁺ T lymphocytes in SS model mice. ^{∗∗}*P*<0.01 vs. Normal; #*P*<0.05 vs. Model
   The results of Fig.6 showed that CP-25 could increase the ratio of CD4⁺ CD25⁺FOXP3⁺ Treg lymphocytes in SS model mice.

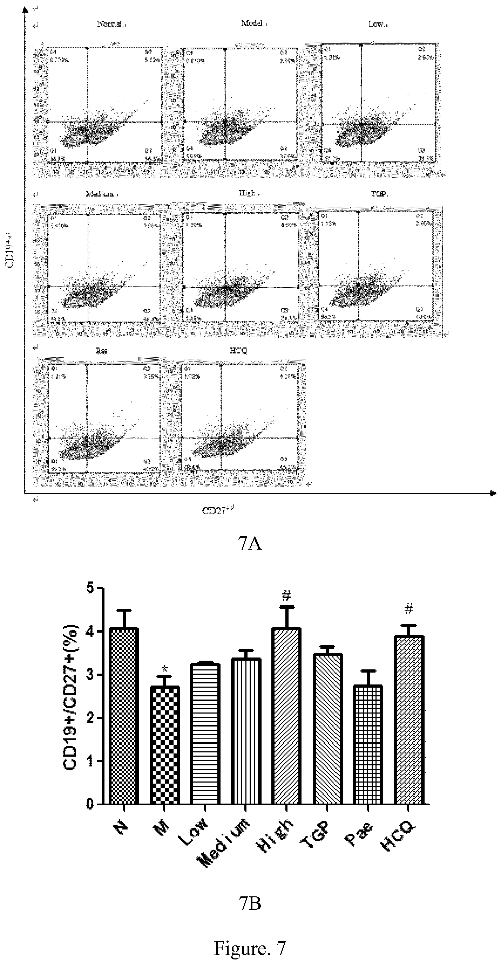

Fig.7 Effects of CP-25 on spleen CD19⁺ CD27⁺ B lymphocytes in SS model mice. ^{∗}*P*<0.05 vs. Normal; # *P*<0.05 vs. Model
   The results of Fig.7 showed that CP-25 could significantly increase the ratio of spleen CD19⁺ CD27⁺ B lymphocytes in SS model mice.

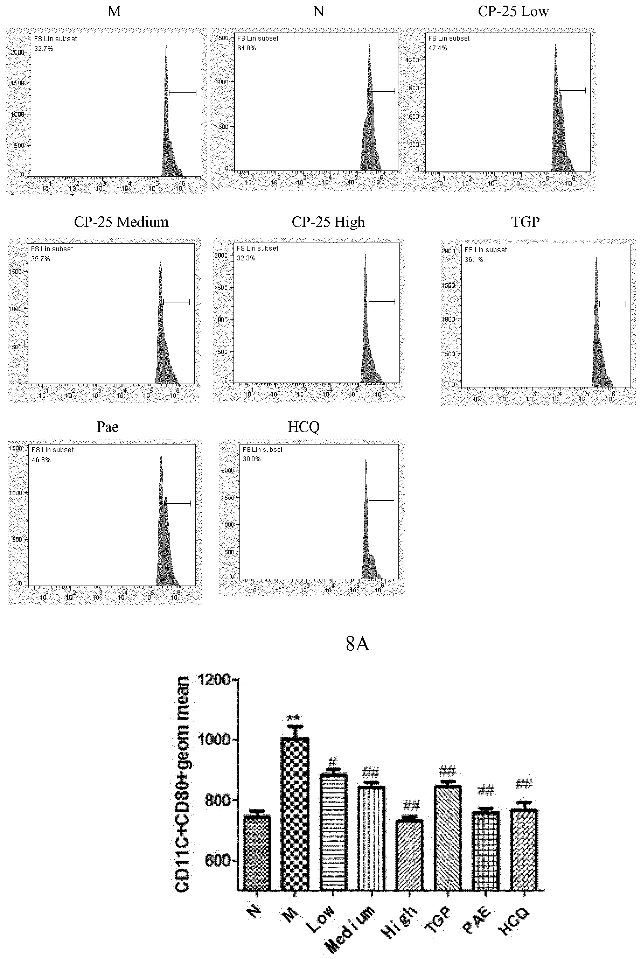

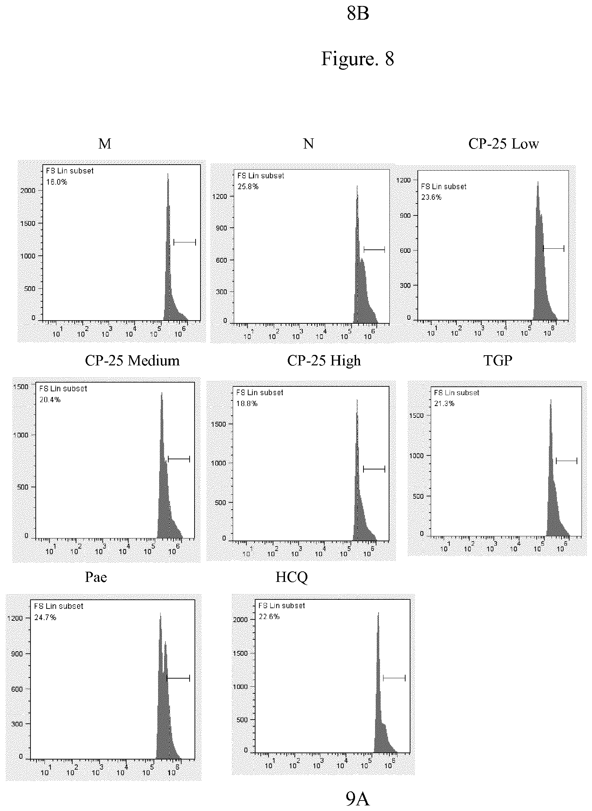

Fig.8 Effects of CP-25 on spleen CD11c⁺ CD80⁺ DC in SS model mice. ^{∗∗}*P*<0.01 vs. Normal; # *P*<0.05, # # *P*<0.01 vs. Model
   The results of Fig.8 showed that CP-25 could significantly inhibit the expression of surface molecular CD80⁺ on spleen CD11c⁺ DC in SS model mice.

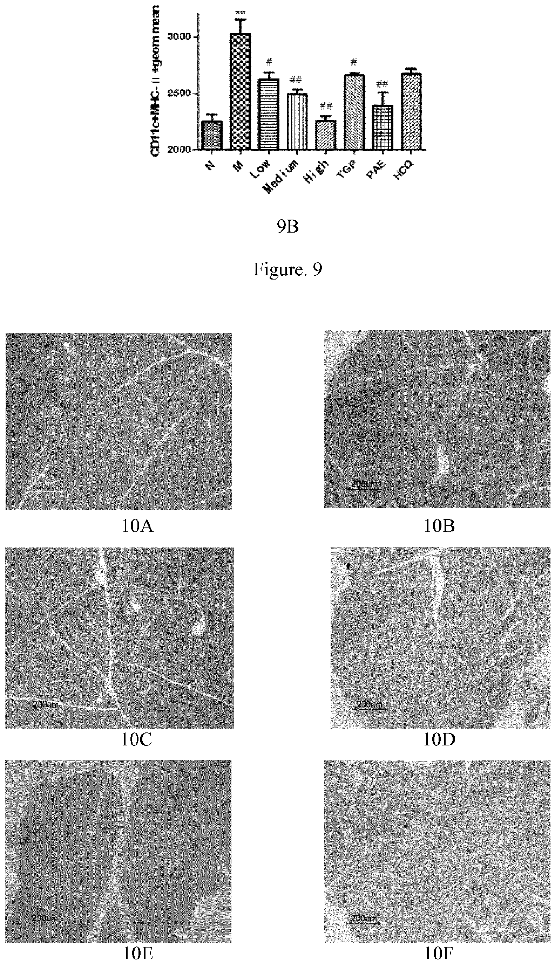

Fig.9 Effects of CP-25 on spleen CD11c⁺ MHC-II⁺ DC in SS model mice. ^{∗∗}*P*<0.01 vs. Normal; # *P*<0.05, # # *P*<0.01 vs. Model
   The results of Fig.9 showed that CP-25 could significantly inhibit the expression of surface molecular MHC-II⁺ on spleen CD11c⁺ DC in SS model mice.

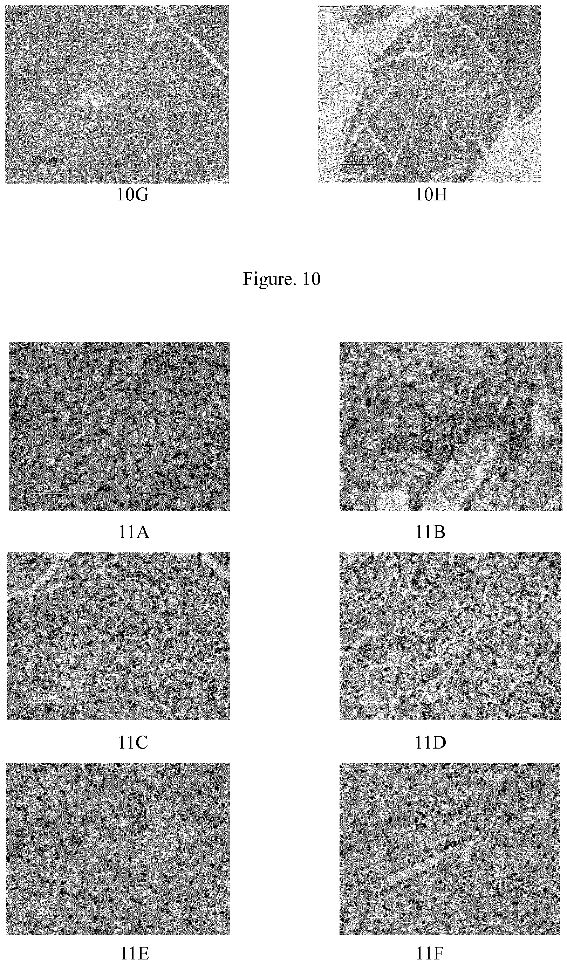

Fig.10 Effects of CP-25 on pathology of submaxillary gland in NOD model mice (HE × 100).
   A: Control; B: Model; C: Low dose of CP-25; D: Medium dose of CP-25; E: High dose of CP-25; F: TGP; G: Pae; H: HCQ.

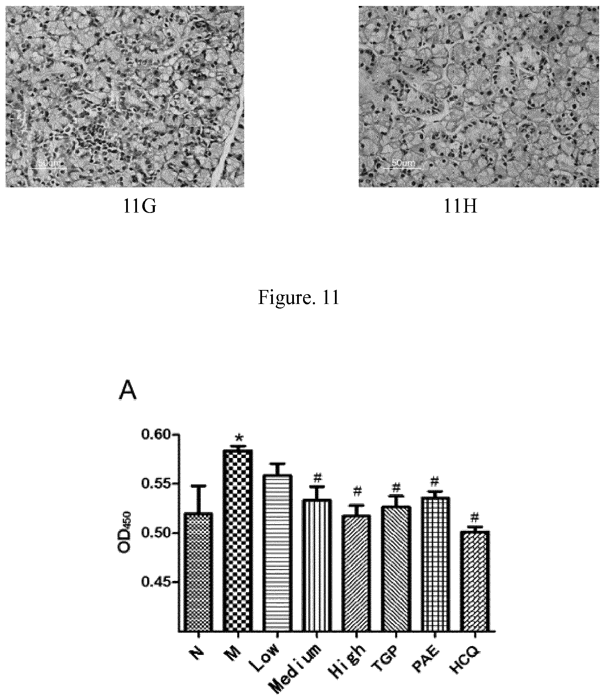

Fig.11 Effects of CP-25 on pathology of submaxillary gland in NOD model mice (HE×400).
   A: Control; B: Model; C: Low dose of CP-25; D: Medium dose of CP-25; E: High dose of CP-25; F: TGP; G: Pae; H: HCQ.

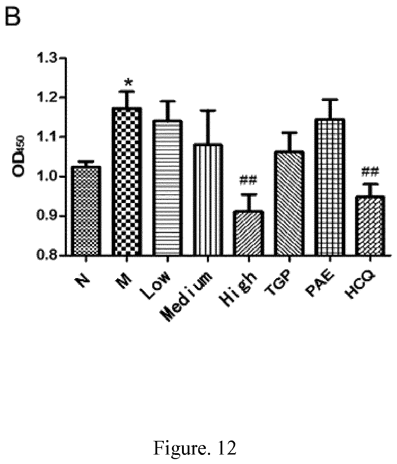

Fig.12 Effects of CP-25 on spleen T and B lymphocyte proliferation in NOD model mice.
   A: T lymphocytes; B: B lymphocytes
   The results of Fig.12 showed that CP-25 could significantly inhibit T and B lymphocyte proliferation in NOD model mice.

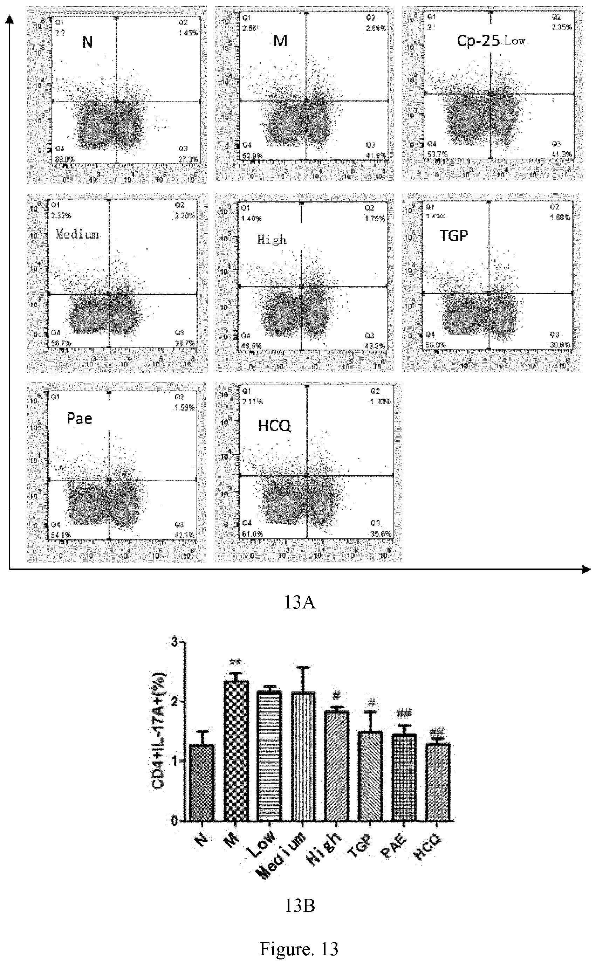

Fig.13 Effects of CP-25 on spleen CD4⁺ IL-17A⁺ T lymphocytes in NOD model mice.
   ^{∗∗}*P*<0.01 vs. Normal; # *P*<0.05, # # *P*<0.01 vs. Model
   The results of Fig.13 showed that CP-25 could significantly inhibit the ratio of spleen CD4⁺IL-17A⁺ Th17 lymphocytes in NOD model mice.

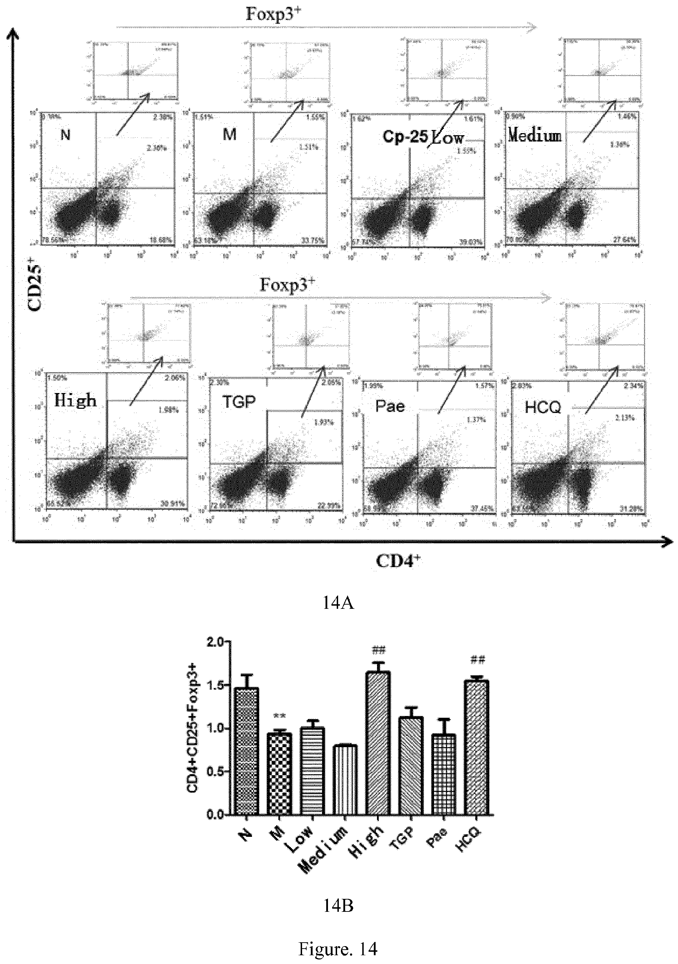

Fig.14 Effects of CP-25 on spleen CD4⁺ CD25⁺FOXP3⁺ T lymphocytes in NOD model mice.
   ^{∗∗}P<0.01 vs. Normal; # # *P*<0.01 vs. Model
   The results of Fig.14 showed that CP-25 could significantly improve the ratio of spleen CD4⁺ CD25⁺FOXP3⁺ Treg lymphocytes in NOD model mice.

### Embodiments

Hereinafter, the invention will be further described with reference to the following examples:
**§ 1. Classified diagnostic criteria for Sjögren's syndrome** (Chin. J. Rheumatol. 2010; 14(11): 766-768):

**Table 1. Items of classification criteria for Sjögren's syndrome**

| | |
|---|---|
| I. Oral symptoms: a positive response to at least one of the following three examination items: | |
| | 1. Having had a daily feeling of dry mouth for more than 3 months; |
| | 2. Having had recurrently or persistently swollen parotid gland as an adult; |
| | 3. Need drinking liquids to aid in swallowing dry food. |
| II. Ocular symptoms: a positive response to at least one of the following three examination items: | |
| | 1. Having daily, intolerable dry eyes for more than 3 months; |
| | 2. Having a recurrent sensation of sand or gravel in the eyes; |
| | 3. Using artificial tears more than 3 times a day. |
| III. Ocular signs-that is, objective evidence of ocular involvement defined as a positive result for at least one of the following two tests: | |
| | 1. Schirmer I test (+) (≤5 mm / 5 minutes) |
| | 2. Corneal staining (+) (≥4 according to van Bijsterveld's scoring system) |
| IV. Histological examination: The pathology of labial gland indicates that, lymphocytic foci≥1, (lymphocytic foci being defined as an aggregate of 50 or more lymphocytes gathered in the labial gland stroma per 4 mm² of tissue. | |
| V. Salivary gland involvement: objective evidence of salivary gland involvement defined by a positive result for at least one of the following diagnostic tests: | |
| | 1. Unstimulated whole salivary flow (+) (≤1.5 ml/15 minutes) |
| | 2. Parotid contrast (+) |
| | 3. Salivary gland radioisotope examination (+) |
| VI. Autoantibodies: Antibodies to SSA or SSB antigens (+) (Double diffusion method) | |

**Table 2 Specific categories of items of classification criteria**

| |
|---|
| 1. For primary SS: in patients without any potentially associated disease, primary SS may be defined as follows: |
| a. The presence of any 4 of the 6 items listed in Table 1. is indicative of primary SS, provided that item IV (Histological examination) and/or VI (Autoantibodies) is positive; |
| b. The presence of any 3 of the 4 objective criteria items, that is, items III, IV, V, VI are positive. |
| 2. For secondary SS: In patients with a potentially associated disease (for instance, another well defined connective tissue disease), the presence of item I or item II plus any 2 from among items III, IV, and V liseted in Table 1. may be considered as indicative of secondary SS. |
| 3. Exclusion criteria: Past head and neck radiation treatment; Hepatitis C infection; Acquired immunodeficiency disease (AIDS); Pre-existing lymphoma Sarcoidosis; Graft versus host disease; Use of anticholinergic drugs (for example, Atropine, Hyoscyamine, Propantheline bromide, Belladonna, etc.) |

| |
|---|
| Wherein: the positive percentage of antinuclear antibody (ANA), rheumatoid factor (RF), anti-SSA/Ro autoantibody, anti-SSB/La autoantibody and hyperimmunoglobulinemia was 92%, 70% ∼ 80%, 70%, 45%, 90%, respectively. |

**§ 2 Sjögren's syndrome model mice:** C57BL/6 female mice housed in a SPF environment with successful modeling (The animal model was made via repeated multi-point intracutaneous injection of submaxillary gland homogenate of homologous mice to tail and back of mice).
**§ 3 Instruments and agents:** analytical balance, heater, eye scissors, ophthalmic forceps, 1ml of syringe; CP-25, sodium carboxymethylcellulose (CMC-Na), 2.4% pentobarbital sodium, 0.025 mg/ml pilocarpine.
**§ 4 Construction of model and grouping:**
The animal model was made via repeated multi-point intracutaneously injection of submaxillary gland homogenate of homologous mice to tail and back of mice, and the mice with successful modeling were randomly assigned to each group according to comprehensive conditions of mice. At the same time, the average weight and status of each group of mice should be as close as possible.
**§ 5 Preparation and administration of paeoniflorin-6'-O-benzenesulfonate:**
Paeoniflorin-6'-O-benzenesulfonate, white crystalline powder, purity 98.5%, was dissolved in trace ethanol (75 mg of CP-25 was soluble in 40 µl of ethanol), and then sodium carboxymethylcellulose was added to formulate a suspension of CP-25. Corresponding dose gradient, low dose (17.5mg/kg), medium dose (35mg/kg) and high dose (70mg/kg) of said pharmaceutical should be prepared before medication, stored in 4°C refrigerator for use. High dose of said pharmaceutical should be incubated for half an hour before use.
**§ 6 Measurement of water intake:**
Water intake in therapeutic mice tends to be increased before medication, but begins to drop off after a week of administration with CP-25. However, the water intake in mice of model control group always tends to rise.
**§ 7 Measurement of saliva amount**
Before measurement, mice were anesthetized via intraperitoneal injection of 0.05 ml of 2.4% pentobarbital sodium, and the satisfactory anesthesia was smooth breathing, corneal reflex disappearance and limb muscle relaxation. After complete anesthesia, a mouse was placed in slightly tilted trendelenburg position, and its body was kept warm using the heater. The mouse was injected intraperitoneally with 0.1 ml of 0.025mg/ml Pilocarpine, and 5 min later, a weighed cotton ball was placed in the mouth of the mouse for 10 min. After taking the ball out, the saliva weight of mice was measured by the wet weight method. Saliva amount in model group of mice was apparently decreased, however, that in therapeutic group of mice was obviously recovered after one and two weeks of administration.

**Table 3. Saliva amount in mice administrated with corresponding drug determined pre-administration, after one and two weeks of administration, respectively (mg/10min)**

| Group | Dose (mg/kg) | Pre-administration | One week after administration | Two weeks after administration |
|---|---|---|---|---|
| Normal | - | 32.33±3.75 | 29.33±2.08 | 29.00±2.00 |
| Model | - | 24.50±3.14^{ΔΔ} | 16.50±2.07^{ΔΔ} | 15.07±3.03^{ΔΔ} |
| CP-25 | 17.5 | 22.67±3.27 | 17.83±2.14 | 17.67±2.07 |
| | 35 | 23.01±2.37 | 18.33±1.97 | 23.17±2.64^{##} |
| | 70 | 22.75±3.67 | 25.67±1.63^{##∗} | 26.83±1.47^{##} |
| TGP | 70 | 23.84±2.64 | 21.00±3.97 | 22.17±2.93^{#} |
| Pae | 70 | 23.50±2.89 | 17.01±2.10 | 17.17±2.72 |
| HCQ | 80 | 23.17±2.79 | 18.17±3.65 | 23.67±225^{##} |

| | | | | |
|---|---|---|---|---|
| ^{ΔΔ}*P*<0.01 vs. Normal; ^{#} *P<0.05,* ^{##}*P*<0.01 vs. Model; ^{∗}*P*<0.05 vs. TGP, Pae and HCQ respectively. | | | | |

The results of saliva amount in mice showed that, for treatment of Sjögren's syndrome, high dose of CP-25 had curative effects after one week of medication, but any of TGP, Pae and HCQ did not show apparent effect at that time; with the time of medication extended to two weeks, CP-25 possessed more significant efficacy. It is concluded that, CP-25 has the advantages of rapid effect and better efficacy in treatment of Sjögren's syndrome.
**§ 8 Histopathological examination of submaxillary glands:**
Mice were sacrificed after two weeks of administration, and then submaxillary glands of four mice of each group were removed and pathologically observed. At first, the submaxillary gland should be fixed in 10% buffered formalin for 24 h, followed by washing, dehydration and clearing, wax-filling, embedding, sectioning and finally H&E staining. There were varying degrees and ingravescence of lymphocyte infiltration in submaxillary glands in model mice; on the contrary, described infiltration could be significantly improved in therapeutic group of mice. Results were graded pathologically.
Note: The histological findings graded of submaxillary glands according to the Chisholm-Mason scale; grade 0, absence of lymphocytic infiltration; grade I, slight lymphocytic infiltration; grade II, moderate lymphocytic infiltration; grade III, one focus of lymphocytic infiltration per 4 mm² tissue; grade IV, more than one focus of lymphocytic infiltration per 4 mm² tissue (Note: One focus of lymphocytic infiltration has been defined as an aggregate of 50 or more lymphocytes gathered in the labial gland stroma per 4 mm² of tissue.

**Table 4. Effects on pathological grading in submaxillary gland in mice of respective groups**

| Group | Dose (mg/kg) | Score | | | | |
|---|---|---|---|---|---|---|
| | | Grade 0 | Grade I | Grade II | Grade III | Grade IV |
| Control | - | 0 | 3 | 1 | 0 | 0 |
| Normal | - | 0 | 0 | 0 | 0 | 4^{ΔΔ} |
| CP-25 | 17.5 | 0 | 0 | 0 | 1 | 3 |
| | 35 | 0 | 0 | 0 | 1 | 3 |
| | 70 | 0 | 0 | 0 | 3 | 1^{#} |
| TGP | 70 | 0 | 0 | 0 | 2 | 2^{#} |
| Pae | 70 | 0 | 0 | 0 | 2 | 2^{#} |
| HCQ | 80 | 0 | 0 | 0 | 3 | 1^{#} |

| | | | | | | |
|---|---|---|---|---|---|---|
| *^{ΔΔ}P*<0.01 *vs.* Normal; *^{#}P*<0.05, ^{##}*P*<0.01 *vs.* Model | | | | | | |

The results of H&E staining score in submaxillary gland by rank-sum test showed that, high dose of CP-25 possessed definitely curative effects on Sjögren's syndrome, and high dose of CP-25 was able to apparently ameliorate the pathological changes in submaxillary glands.

### Example 1

The therapeutic action of CP-25 on induced Sjögren's syndrome in C57BL/6 mice.

**Table 5. The effects of CP-25 on saliva amount in SS mice (mg/each mouse/10 min)**

| Group | Dose (mg/kg) | Pre-administration | One week after administration | Two weeks after administration |
|---|---|---|---|---|
| Normal | - | 32.33±3.75 | 29.33±2.08 | 29.00±2.00 |
| Model | - | 24.50±3.14^{ΔΔ} | 16.50±2.07^{ΔΔ} | 15.07±3.03^{ΔΔ} |
| CP-25 | 17.5 | 22.67±3.27 | 17.83±2.14 | 17.67±2.07 |
| | 35 | 23.01±2.37 | 18.33±1.97 | 23.17±2.64^{##} |
| | 70 | 22.75±3.67 | 25.67±1.63^{##∗} | 26.83±1.47^{##} |
| TGP | 70 | 23.84±2.64 | 21.00±3.97 | 22.17±2.93^{#} |
| Pae | 70 | 23.50±2.89 | 17.01±2.10 | 17.17±2.72 |
| HCQ | 80 | 23.17±2.79 | 18.17±3.65 | 23.67±2.25^{##} |

| | | | | |
|---|---|---|---|---|
| **Note:** ^{ΔΔ}*P*<0.01 vs. Normal; *^{#}P*<0.05, ^{##}*P*<0.01 vs. Model; ^{∗}*P*<0.05 vs. TGP, Pae and HCQ respectively. | | | | |

CP-25 could significantly increase the saliva amount in Sjögren's syndrome mice, and the onset time of curative effects of CP-25 was earlier than that of TGP, Pae or HCQ correspondingly.

### Example 2

**Table 6. The effect of CP-25 on organic indexes in SS mice (mg/g)**

| Group | Dose (mg/kg) | Spleen index | Submaxillary gland index | Thymus index |
|---|---|---|---|---|
| Normal | - | 6.51±1.10 | 3.42±0.44 | 0.82±0.12 |
| Model | - | 9.93±1.41^{Δ} | 4.33±0.51^{Δ} | 1.21±0.23^{Δ} |
| CP-25 | 17.5 | 9.00±1.61 | 3.56±0.62 | 1.06±0.31 |
| | 35 | 8.92±1.33 | 3.32±0.66^{#} | 1.31±0.42 |
| | 70 | 8.51±1.42 | 3.24±0.57^{#} | 1.03±0.44 |
| TGP | 70 | 8.33±1.36 | 2.86±0.53^{##} | 1.12±0.37 |
| Pae | 70 | 9.15±1.44 | 3.71±0.68 | 0.85±0.23 |
| HCQ | 80 | 9.03±1.71 | 3.52±0.54 | 0.89±0.31 |

| | | | | |
|---|---|---|---|---|
| **Note:** ^{Δ}*P*<0.05 vs. Normal; *^{#}P*<0.05, ^{##}*P*<0.01 vs. Model | | | | |

CP-25 could significantly suppress the increasing of submaxillary gland index in SS mice.

### Example 3

**Table 7. Effects of CP-25 on pathological grading in submaxillary glands in SS mice**

| Group | Dose (mg/kg) | Score | | | | |
|---|---|---|---|---|---|---|
| | | Grade 0 | Grade I | Grade II | Grade III | Grade IV |
| Control | - | 0 | 3 | 1 | 0 | 0 |
| Normal | - | 0 | 0 | 0 | 0 | 4^{ΔΔ} |
| CP-25 | 17.5 | 0 | 0 | 0 | 1 | 3 |
| | 35 | 0 | 0 | 0 | 1 | 3 |
| | 70 | 0 | 0 | 0 | 3 | 1^{#} |
| TGP | 70 | 0 | 0 | 0 | 2 | 2^{#} |
| Pae | 70 | 0 | 0 | 0 | 1 | 3 |
| HCQ | 80 | 0 | 0 | 0 | 3 | 1^{#} |

| | | | | | | |
|---|---|---|---|---|---|---|
| ^{ΔΔ}*P*<0.01 *vs.* Normal; #*P*<0.05 *vs.* Model | | | | | | |

CP-25 was able to apparently ameliorate the pathological changes in submaxillary gland in SS mice.

### Example 4

Therapeutic effects of on Sjögren's syndrome CP-25 in NOD mice.

**Table 8. The effects of CP-25 on saliva amount in NOD mice (mg/each mouse/10 min)**

| Group | Dose (mg/kg) | Pre-administration | One week after administration | Two weeks after administration |
|---|---|---|---|---|
| Normal | - | 26.43±4.79 | 27.86±2.91 | 28.50±2.17 |
| Model | - | 20.00±1.79^{∗} | 15.17H.57^{∗∗} | 12.50±1.30^{∗∗} |
| CP-25 | 17.5 | 19.50±1.38 | 15.20±1.04 | 14.25±1.46 |
| | 35 | 19.33±3.20 | 15.60±1.50 | 15.25±1.36^{#} |
| | 70 | 19.51±1.87 | 18.20±1.58^{#Δ} | 18.25±1.50^{##} |
| TGP | 70 | 20.00±3.89 | 16.10±1.52 | 16.00±1.50^{#} |
| Pae | 70 | 19.83±1.94 | 15.40±1.62 | 14.75±1.25 |
| HCQ | 80 | 19.17±3.18 | 17.00±1.61^{#} | 17.00±1.41^{##} |

| | | | | |
|---|---|---|---|---|
| ^{∗}*P*<0.05*,* ^{∗∗}*P*<0.01 vs. Normal; *^{#}P*<0.05, ^{##}*P*<0.01 vs. Model; ^{Δ}*P*<0.05 vs.TGP and Pae respectively. | | | | |

CP-25 could significantly increase the saliva amount in model mice, and the onset time of curative effects of CP-25 was earlier than that of TGP, or Pae.

### Example 5

**Table 9. The effect of CP-25 on organic indexes in NOD model mice (mg/g)**

| Group | Dose (mg/kg) | Spleen index | Submaxillary gland index | Thymus index |
|---|---|---|---|---|
| Normal | - | 4.5±1.5 | 4.0±0.4 | 2.5±1.2 |
| Model | - | 5.3±0.6 | 5.6±0.5^{∗} | 0.7±0.2 |
| CP-25 | 17.5 | 9.5±4.2 | 4.5±0.8 | 1.3±0.7 |
| | 35 | 4.2±0.6 | 4.3±0.6^{#} | 1.1±0.6 |
| | 70 | 4.3±0.7 | 4.2±0.6^{#} | 1.4±0.5 |
| TGP | 70 | 5.2±0.9 | 4.3±0.2^{#} | 1.0±0.3 |
| Pae | 70 | 5.3±0.5 | 4.5±0.5 | 1.0±0.1 |
| HCQ | 80 | 4.1±0.6 | 4.2±0.8 ^{#} | 1.3±0.6 |

| | | | | |
|---|---|---|---|---|
| ^{∗}*P*<0.05 vs. Normal; #*P*<0.05 vs. Model. | | | | |

CP-25 could significantly suppress the increasing of submaxillary gland index in model mice.

### Example 6

**Table 10. Effects of CP-25 on pathological grading in submaxillary glands in NOD**

| mice | | | | | | |
|---|---|---|---|---|---|---|
| Group | Dose (mg/kg) | Score | | | | |
| | | Grade 0 | Grade I | Grade II | Grade III | Grade IV |
| Control | - | 0 | 2 | 1 | 0 | 0 |
| Normal | - | 0 | 0 | 0 | 1 | 3^{∗} |
| CP-25 | 17.5 | 0 | 0 | 0 | 1 | 3 |
| | 35 | 0 | 0 | 0 | 1 | 3 |
| | 70 | 0 | 0 | 0 | 3 | 1 ^{#} |
| TGP | 70 | 0 | 0 | 0 | 2 | 2 |
| Pae | 70 | 0 | 0 | 0 | 1 | 3 |
| HCQ | 80 | 0 | 0 | 0 | 3 | 1 ^{#} |

| | | | | | | |
|---|---|---|---|---|---|---|
| ^{∗}*P*<0.01 *vs.* Normal; #*P*<0.05 *vs.* Model | | | | | | |

CP-25 was able to apparently ameliorate the pathological changes in submaxillary gland in NOD model mice.

### Analysis and discussion

According to examples described above, the present inventors analysed the experimental data and considered that: High dose of CP-25 showed more rapid effects than medium dose or low dose of CP-25 in some therapeutic indexes (for example, the effects of CP-25 on saliva amount in SS mice (mg/10 min), see Table 5), and showed comparable efficacy to medium dose or low dose of CP-25 in other indexes (for example, The effect of CP-25 on organic index in SS mice (mg/g), see Table 6). Considering that the Sjögren's syndrome is characterised of relatively slow progress and relatively long course, the patients with Sjögren's syndrome, differing from patients with other diseases, generally expect to be treated with small dosage and good effect. Meantime, the patients with Sjögren's syndrome have higher expectation on persistence of efficacy. Base on the curative characterization above-mentioned of the disease and patients, medium dose and low dose of CP-25 possess significantly substantial advantages and notable progression in the treatment of Sjögren's syndrome as compared with high dose of CP-25, TGP, Pae or HCQ. To summarize, high dose and medium dose of CP-25 could be used in a short-term in the initial stage of treatment of Sjögren's syndrome, following by long-term use of medium dose and low dose of CP-25. Preferably, high dose of CP-25 could be used in a short-term in the initial stage of treatment of Sjögren's syndrome, and then use of medium dose of CP-25 in a short-term, following by long-term use of low dose of CP-25. More preferably, medium dose of CP-25 could be used in a short-term in the initial stage of treatment of Sjögren's syndrome, following by long-term use of low dose of CP-25. Most preferably, low dose of CP-25 is used to cure Sjögren's syndrome in the whole course of treatment.

### Drug combination

The persons skilled in this art can easily conceive that: CP-25 could be used as an active ingredient in a drug combination for the treatment of Sjögren's syndrome in combination with drug for autoimmune diseases. Said drug for autoimmune diseases includes, but is not limited to, biological agents acting on TNF-α taget, for example, Humira, Enbrel, Remicade, Simponi (golimumab), Cimzia (Certolizumab pegol), Etanercept (Recombinant human tumor necrosis factor-α receptor II: IgG Fc fusion protein for injection); other biological agents for the treatment of autoimmune diseases, for example, Actemra (Ocilizumab), Orencia (Abatacept), Stelara (ustekinumab); JAK inhibitors acting on JAK1, JAK2, JAK3 or TYK2 target, etc., for example, Xeljanz (Tofacitinib), Baricitinib; selective phosphodiesterase 4 (PDE4) inhibitors, for example, Otezla (Apremilast).

Similarly, the persons skilled in this art can easily conceive that: CP-25 could also be used as an active ingredient in a drug combination for the treatment of Sjögren's syndrome in combination with the further drug for the treatment of Sjögren's syndrome. Said the further drug for the treatment of Sjögren's syndrome includes, but is not limited to, drugs for relieving local dryness symptom, for example, Pilocarpine, Ciclosporin, Anethol Trithione, Cevimeline, artificial tears; immunosuppressive agents for systemic therapy, for example, Methotrexate, Leflunomide, Cyclophosphamide, Azathioprine, Tripterygium wilfordii, Tacrolimus, Mycophenolate mofetil; glucocorticoids, for example, Prednisone, Methylprednisolone; and other drugs for treatment of autoimmune diseases, for example, Hydroxychloroquine, Sulfasalazine, anti CD20 antibody.

The further therapeutic agent described in this invention comprises a drug for promoting treatment of Sjögren's syndrome, including but not limited to, one or more members selected from the group consisting of the substances described in the following patent application/patent:

**Table 11. Some patents/applications related to the further therapeutic agents as active ingredients in drug combinations**

| **Patent/Publication No.** | **Title of invention** |
|---|---|
| WO2016204429 (A1) | Heteroaryl derivative or pharmaceutically acceptable salt thereof, preparation method therefor, and pharmaceutical composition for preventing or treating diseases associated with pi3 kinases, containing same as active ingredient |
| CA2950893 (A1) | Novel combinations for antigen based therapy |
| KR20160126734 (A) | A method for producing autoimmune disease patient-specific induced pluripotent stem cell and a use thereof |
| HK1216994 (A1) | Reducing the risk of autoimmune disease |
| US2016362462 (A1) | Modification and Novel Compositions of Human Secretoglobin Proteins |
| US2016361407 (A1) | Recombinant lactococcus lactis expressing escherichia coli colonization factor antigen i (cfa/i) fimbriae and their methods of use |
| US2016361360 (A1) | Disease therapy with chimeric antigen receptor (car) constructs and t cells (car-t) or nk cells (car-nk) expressing car constructs |
| US2016361428 (A1) | Metap-2 inhibitor polymersomes for therapeutic administration |
| US2016361300 (A1) | Use of telmisartan to prevent and treat graft versus host disease and other alloimmune and autoimmune diseases |
| WO2016200447 (A1) | Nicotinamide riboside and pterostilbene compositions and methods for treatment of skin disorders |
| CA2950423 (A1) | Compositions and methods relating to universal glycoforms for enhanced antibody efficacy |
| US2016348072 (A1) | Activation and Expansion of T-Cells Using an Engineered Multivalent Signaling Platform as a Research Tool |
| US2015065352 (A1); US9512487 (B2) | Monitoring health and disease status using clonotype profiles |
| US2016356788 (A1) | Methods and compositions for diagnosis and treatment of autoimmune disease secondary to multiple sclerosis |
| US2016355591 (A1) | Subcutaneous anti-hla-dr monoclonal antibody for treatment of hematologic malignancies |
| US2016355464 (A1) | Fumarate compounds, pharmaceutical compositions thereof, and methods of use |
| US2016354437 (A1) | Compositions and methods for modulation of immune response |
| TW201632559 (A) | Antibody therapeutics that bind CD137 |
| TW201630938 (A) | Novel anti-CD38 antibodies for the treatment of cancer |
| TW201630880 (A) | Indole carboxamide compounds |
| WO2016196429 (A1) | An engineered ccl20 locked dimer polypeptide |
| WO2016196393 (A2) | Autophagy-inhibiting compounds and uses thereof |
| MD20160069 (A2) | Apotosis signal-regulating kinase inhibitors |
| CA2949966 (A1) | Treatment of autoimmune disease |
| US2016346328 (A1) | Somatic stem cells |
| US2016346355 (A1) | Cytokine receptor peptides, compositions thereof and methods thereof |
| US2016345547 (A1) | Non-human animals having a disruption in a c9orf72 locus |
| NZ702458 (A) | Benzo [c] isoxazoloazepine bromodomain inhibitors and uses thereof |
| WO2016191545 (A1) | Personalized delivery vector-based immunotherapy and uses thereof |
| WO2016190630 (A1) | Heterocyclicalkyl derivative compounds as selective histone deacetylase inhibitors and pharmaceutical compositions comprising the same |
| WO2016191634 (A1) | Methods of preventing and treating autoimmunity |
| AU2015253915 (A1) | Antibody binding to fcrn for treating autoimmune diseases |
| AU2015244025 (A1) | Binding molecules specific for IL-21 and uses thereof |
| AU2015254818 (A1) | Variants of DR3 and use thereof |
| AU2015254037 (A1) | Novel compound having immune disease treatment effect and use thereof |
| US2016339053 (A1) | Compositions and Methods for Treating Autoimmune and Inflammatory Diseases |
| US2016339049 (A1) | Use of cladribine for treating autoimmune inflammatory disease |
| US2016340337 (A1) | Benzoimidazol-2-yl pyrimidine modulators of the histamine h4 receptor |
| US2016338984 (A1) | Particle formulations of all-trans retinoic acid and transforming growth factor beta for the treatment of type 1 diabetes mellitus |
| US2016338953 (A1) | Liposome-based immunotherapy |
| WO2016185476 (A1) | Methods of obtaining mononuclear blood cells and uses thereof |
| HRP20161191 (T1) | Redirected, genetically-engineered t regulatory cells and their use in suppression of autoimmune and inflammatory disease |
| RU2601410 (C1) | {3-[(7H-PYRROLO[2,3-d]PYRIMIDIN-4-YL)AZOLYL]AZETIDIN-3-YL} ACETONITRILES AS JANUS KINASE INHIBITORS |
| US2016333089 (A1) | Antigenic GM-CSF Peptides and Antibodies to GM-CSF |
| US2016333103 (A1) | Baff selective binding compounds and related methods |
| US2016333409 (A1) | Method for identifying disease-associated cdr3 patterns in an immune repertoire |
| US2016331834 (A1) | Epicutaneous immunorebalancing |
| US2016331816 (A1) | Oral delivery of angiotensin converting enzyme 2 (ace2) or angiotensin-(1-7) bioencapsulated in plant cells attenuates pulmonary hypertension, cardiac dysfunction and development of autoimmune and experimentally induced ocular disorders |
| AU2016250372 (A1) | Immune regulatory oligonucleotide (IRO) compounds to modulate toll-like receptor based immune response |
| AU2016247182 (A1) | Substituted pyridine-2-carboxamide compounds as apoptosis signal-regulating kinase inhibitors |

### Dose for human use and therapeutically effective dose

### Dose of CP-25

In this invention, the dose of CP-25 in animal experiment is 17.5, 35, or 70mg/kg/day respectively, which was the usual dose in animal experiment. According to the ratio of equivalent dose to body surface area between human and animals, the corresponding dose for human adult is about 1.75 mg/kg/day to 7.0 mg/kg/day. Thus, about 105 mg to 420 mg of CP-25 is taken per time for a 60 kg human patient, and about 315 mg to 1260 mg of CP-25 for human adult is taken per day if taken 3 times daily.

As described above, according to the teaching of the present invention, those skilled in the art could easily conceive that, said dose means the effective dose, that is, the dose which can effective cure and/or prevent said Sjögren's syndrome, wherein including the dose of active ingredient of CP-25, the total dose of combination of CP-25 and above-mentioned further drugs or other agents, and the respective dose in the drug combination of CP-25 and above-mentioned further drugs or other agents. By reading this specification of the present invention, those skilled in the art will know that, the effective dose of the invention is an important component of the invention, and constitutes the present invention along with other parts of the invention.

### Pharmaceutical preparations

The pharmaceutical compositions of this invention could be prepared according to the methods known for persons skilled in the art. Said compositions can comprise effective dose of CP-25 based on this invention, effective dose of above-mentioned further drugs or drug combination of other agents, and supporting agent and excipient of appropriate carriers or mediators as the active ingredient. Said supporting agent or excipient is known in this field. The compositions are preferably used as oral agents. The compositions may be administered in various dosage forms, preferably in the form of tablets or capsules. Other dosage forms, such as suppositories, solution, suspensions, dry suspensions, syrup, etc., are optionally used. Many sustained-release technologies and preparations are known in the art, and can be used in this invention. A preparation with multiple effects in combination with sustained-release and gastric protective actions also is optional, and can be used in this invention.

## Claims

1. Paeoniflorin-6'-O-benzenesulfonate for use in increasing saliva flow in mammalian Sjögren's syndrome.

2. Paeoniflorin-6'-O-benzenesulfonate for use as claimed in claim 1, **characterized in that** the effective amount of paeoniflorin-6'-O-benzenesulfonate ranges from 17.5mg/kg to 70mg/kg.

3. Paeoniflorin-6'-O-benzenesulfonate for use as claimed in claim 1, **characterized in that** the effective amount of paeoniflorin-6'-O-benzenesulfonate ranges from 17.5mg/kg to 35mg/kg.

4. Paeoniflorin-6'-O-benzenesulfonate for use as claimed in claim 1, **characterized in that** the effective amount of paeoniflorin-6'-O-benzenesulfonate is 17.5mg/kg.

5. Paeoniflorin-6'-O-benzenesulfonate for use as claimed in any one of claims 1 to 4, wherein said pharmaceutical agent is a drug combination comprising paeoniflorin-6'-O-benzenesulfonate and a further therapeutic agent.

6. Paeoniflorin-6'-O-benzenesulfonate for use as claimed in claim 5, wherein the further therapeutic agent is one or more members selected from the group consisting of: a drug for autoimmune diseases, a further drug for the treatment of Sjögren's syndrome, and a drug for promoting treatment of Sjögren's syndrome.

7. Paeoniflorin-6'-O-benzenesulfonate for use as claimed in claim 6, wherein said drug for autoimmune diseases is one or more members selected from the group consisting of: biological agents acting on TNF-α target, for example, Humira, Enbrel, Remicade, Simponi (Golimumab), Cimzia (Certolizumab pegol), Etanercept (Recombinant human tumor necrosis factor-α receptor II: IgG Fc fusion protein for injection); other biological agents for treatment of autoimmune diseases, for example, Actemra (Ocilizumab), Orencia (Abatacept), Stelara (ustekinumab); JAK inhibitors acting on JAK1, JAK2, JAK3 or TYK2 target, etc., for example, Xeljanz (Tofacitinib), Baricitinib; selective phosphodiesterase 4 (PDE4) inhibitors, for example, Otezla (Apremilast).

8. Paeoniflorin-6'-O-benzenesulfonate for use as claimed in claim 6, wherein the further drug for the treatment of Sjögren's syndrome is one or more members selected from the group consisting of: drugs for relieving local dryness symptom, for example, Pilocarpine, Ciclosporin, Anethol Trithione, Cevimeline, artificial tears; immunosuppressive agents for systemic therapy, for example, Methotrexate, Leflunomide, Cyclophosphamide, Azathioprine, Tripterygium wilfordii, Tacrolimus, Mycophenolate mofetil; glucocorticoids, for example, Prednisone, Methylprednisolone; and other drugs for treatment of autoimmune diseases, for example, Hydroxychloroquine, Sulfasalazine, anti CD20 antibody.

9. Paeoniflorin-6'-O-benzenesulfonate for use as claimed in claim 5, wherein said drug combination is administrated with a composition comprising paeoniflorin-6'-O-benzenesulfonate and the further therapeutic agent, or by respectively administering paeoniflorin-6'-O-benzenesulfonate and the further therapeutic agent, simultaneously or sequentially.

## Patentansprüche

1. Paeoniflorin-6'-O-Benzolsulfonat zur Verwendung zur Steigerung von Speichelfluss bei Sjögren-Syndrom bei Säugetieren.

2. Paeoniflorin-6'-O-Benzolsulfonat zur Verwendung nach Anspruch 1, **dadurch gekennzeichnet, dass** die effektive Menge an Paeoniflorin-6'-O-Benzolsulfonat von 17,5 mg/kg bis 70 mg/kg reicht.

3. Paeoniflorin-6'-O-Benzolsulfonat zur Verwendung nach Anspruch 1, **dadurch gekennzeichnet, dass** die effektive Menge an Paeoniflorin-6'-O-Benzolsulfonat von 17,5 mg/kg bis 35 mg/kg reicht.

4. Paeoniflorin-6'-O-Benzolsulfonat zur Verwendung nach Anspruch 1, **dadurch gekennzeichnet, dass** die effektive Menge an Paeoniflorin-6'-O-Benzolsulfonat 17,5 mg/kg beträgt.

5. Paeoniflorin-6'-O-Benzolsulfonat zur Verwendung nach einem der Ansprüche 1 bis 4, wobei das pharmazeutische Mittel eine Arzneimittelkombination ist, die Paeoniflorin-6'-O-Benzolsulfonat und ein weiteres Heilmittel umfasst.

6. Paeoniflorin-6'-O-Benzolsulfonat zur Verwendung nach Anspruch 5, wobei das weitere therapeutische Mittel eines oder mehrere Elemente ausgewählt aus der Gruppe bestehend aus: einem Arzneimittel für Autoimmunkrankheiten, einem weiteren Arzneimittel zur Behandlung von Sjögren-Syndrom und einem Medikament zur unterstützenden Behandlung von Sjögren-Syndrom ist.

7. Paeoniflorin-6'-O-Benzolsulfonat zur Verwendung nach Anspruch 6, wobei das Arzneimittel für Autoimmunkrankheiten eines oder mehrere Elemente ausgewählt aus der Gruppe bestehend aus: biologischen Mitteln, die auf TNF-α Targets einwirken, zum Beispiel Humira, Enbrel, Remicade, Simponi (Golimumab), Cimzia (Certolizumab pegol), Etanercept (Rekombinanter menschlicher Tumornekrosefaktor-a Rezeptor II: IgG Fc Fusionsprotein zur Injektion); weiteren biologischen Mitteln zur Behandlung von Autoimmunkrankheiten, zum Beispiel Actemra (Tocilizumab), Orencia (Abatacept), Stelara (Ustekinumab); JAK-Inhibitoren, die auf JAK1, JAK2, JAK3 oder TYK2 Targets einwirken, etc., zum Beispiel Xeljanz (Tofacitinib), Baricitinib; selektive Phosphodiesterase-4 (PDE4) Inhibitoren, zum Beispiel Otezla (Apremilast), ist.

8. Paeoniflorin-6'-O-Benzolsulfonat zur Verwendung nach Anspruch 6, wobei das weitere Arzneimittel zur Behandlung von Sjögren-Syndrom eines oder mehrere Elemente ausgewählt aus der Gruppe bestehend aus: Arzneimitteln zur Linderung des Symptoms der lokalen Trockenheit, zum Beispiel Pilocarpin, Ciclosporin, Anetholtrithion, Cevimeline, künstliche Tränen; immunsuppressive Mittel zur systemischen Therapie, zum Beispiel Methotrexat, Leflunomid, Cyclophosphamid, Azathioprin, Tripterygium wilfordii, Tacrolimus, Mycophenolatmofetil; Glukokortikoide, zum Beispiel Prednison, Methylprednisolon; und andere Medikamente zur Behandlung von Autoimmunkrankheiten, zum Beispiel Hydroxychloroquin, Sulfasalazin, Anti-CD20-Antikörper, ist.

9. Paeoniflorin-6'-O-Benzolsulfonat zur Verwendung nach Anspruch 5, wobei die Arzneimittelkombination verabreicht wird mit einer Zusammensetzung, die Paeoniflorin-6'-O-Benzolsulfonat und das weitere therapeutische Mittel umfasst, oder durch das Verabreichen von jeweils Paeoniflorin-6'-O-Benzolsulfonat und dem weiteren therapeutischen Mittel gleichzeitig oder nacheinander.

## Revendications

1. Paéoniflorine-6'-O-benzènesulfonate pour utilisation pour augmenter le flux de salive dans le syndrome de Sjögren mammalien.

2. Paéoniflorine-6'-O-benzènesulfonate pour utilisation selon la revendication 1, **caractérisé en ce que** la quantité efficace de paéoniflorine-6'-O-benzènesulfonate va de 17,5 mg/kg à 70 mg/kg.

3. Paéonifloiine-6'-O-benzènesulfonate pour utilisation selon la revendication 1, **caractérisé en ce que** la quantité efficace de paéoniflorine-6'-O-benzènesulfonate va de 17,5 mg/kg à 35 mg/kg.

4. Paéonifloiine-6'-O-benzènesulfonate pour utilisation selon la revendication 1, **caractérisé en ce que** la quantité efficace de paéoniflorine-6'-O-benzènesulfonate est de 17,5 mg/kg.

5. Paéonifloiine-6'-O-benzènesulfonate pour utilisation selon l'une quelconque des revendications 1 à 4, dans lequel ledit agent pharmaceutique est une combinaison médicamenteuse comprenant du paéoniflorine-6'-O-benzènesulfonate et un autre agent thérapeutique.

6. Paéonifloiine-6'-O-benzènesulfonate pour utilisation selon la revendication 5, dans lequel l'autre agent thérapeutique est un ou plusieurs éléments choisis dans le groupe constitué par : un médicament pour les maladies auto-immunes, un autre médicament pour le traitement du syndrome de Sjögren, et un médicament pour favoriser le traitement du syndrome de Sjögren.

7. Paéonifloiine-6'-O-benzènesulfonate pour utilisation selon la revendication 6, dans lequel ledit médicament pour les maladies auto-immunes est un ou plusieurs éléments choisis dans le groupe constitué par : les agents biologiques agissant sur la cible TNF-α, par exemple, Humira, Enbrel, Remicade, Simponi (golimumab), Cimzia (certolizumab pégol), Etanercept (récepteur II du facteur α de nécrose tumorale humain recombinant : protéine de fusion IgG Fc pour injection) ; d'autres agents biologiques pour le traitement de maladies auto-immunes, par exemple, Actemra (tocilizumab), Orencia (Abatacept), Stelara (ustekinumab) ; les inhibiteurs de JAK agissant sur la cible JAK1, JAK2, JAK3 ou TYK2, etc, par exemple, Xeljanz (tofacitinib), Baricitinib ; les inhibiteurs sélectifs de la phosphodiestérase 4 (PDE4), par exemple, Otezla (Aprémilast).

8. Paéonifloiine-6'-O-benzènesulfonate pour utilisation selon la revendication 6, dans lequel l'autre médicament pour le traitement du syndrome de Sjögren est un ou plusieurs éléments choisis dans le groupe constitué par : les médicaments pour soulager le symptôme de sécheresse locale, par exemple, Pilocarpine, Ciclosporine, Anéthol trithione, Céviméline, les larmes artificielles ; les agents immunosuppresseurs pour thérapie systémique, par exemple, Méthotrexate, Léflunomide, Cyclophosphamide, Azathioprine, Tripterygium wilfordii, Tacrolimus, Mycophénolate mofetil ; les glucocorticoïdes, par exemple, Prednisone, Méthylprednisolone ; et d'autres médicaments pour le traitement de maladies auto-immunes, par exemple, Hydroxychloroquine, Sulfasalazine, un anticorps anti CD20.

9. Paéonifloiine-6'-O-benzènesulfonate pour utilisation selon la revendication 5, dans lequel ladite combinaison médicamenteuse est administrée avec une composition comprenant du paéoniflorine-6'-O-benzènesulfonate et l'autre agent thérapeutique, ou en administrant respectivement du paéoniflorine-6'-O-benzènesulfonate et l'autre agent thérapeutique, simultanément ou séquentiellement.
